(19) Europäisches Patentamt
European Patent Office
Office européen des brevets

(11) Veröffentlichungsnummer: **0 011 818**
**B1**

(12) # EUROPÄISCHE PATENTSCHRIFT

(45) Veröffentlichungstag der Patentschrift: **19.01.83**

(21) Anmeldenummer: **79104635.2**

(22) Anmeldetag: **21.11.79**

(51) Int. Cl.³: **A 61 K 45/06,** A 61 K 31/57, A 61 K 31/63, A 61 K 31/635 // (A61K31/57, 31/54, 31/19, 31/195, 31/38),(A61K31/63, 31/57),(A61K31/635, 31/57)

(54) **Arzneimittel bzw. pharmazeutische Zusammensetzungen auf der Basis von Aldosteronantogonisten und Diuretica sowie Verfahren zu ihrer Herstellung.**

(30) Priorität: **22.11.78 CH 11980/78**

(43) Veröffentlichungstag der Anmeldung: **11.06.80 Patentblatt 80/12**

(45) Bekanntmachung des Hinweises auf die Patenterteilung: **19.01.83 Patentblatt 83/3**

(84) Benannte Vertragsstaaten: **AT BE CH DE FR GB IT LU NL SE**

(56) Entgegenhaltungen:
**DE-A-2 823 239**
**GB-A-1 041 534**
**CHEMICAL ABSTRACTS, Band 66, Nr. 13, 27. März 1967, Seite 5089, Zusammenfassung Nr. 54073g. Columbus, Ohio, USA L. DETTLI et al. »Therapy with combinations of diuretic agents; comparative studies«.**
**CHEMICAL ABSTRACTS, Band 54, Nr. 21, 10. November 1960, Zusammenfassung 23057h. Columbus, Ohio, USA C. J. EDMONDS et al. »Action of hydroflumethiazide in relation to adrenal steroids and potassium loss«.**

(73) Patentinhaber: **CIBA-GEIGY AG, Patentabteilung Postfach, CH-4002 Basel (CH)**

(72) Erfinder: **Biollaz, Michel, Dr., Morystrasse 2, CH-4125 Riehen (CH)**

(74) Vertreter: **Zumstein, Fritz sen., Dr. et al, Bräuhausstrasse 4, D-8000 München 2 (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 011 818 B1

BUNDESDRUCKEREI BERLIN

# Arzneimittel bzw. pharmazeutische Zusammensetzungen auf der Basis von Aldosteronantagonisten und Diuretica sowie Verfahren zu ihrer Herstellung

Die vorliegende Erfindung betrifft in erster Linie neue kaliumsparende Arzneimittel auf der Basis von Aldosteronantagonisten und Diuretika mit diuretischer und/oder saluretischer Wirkung, die zugleich eine verminderte Ausscheidung von Kalium aufweisen. Nachstehend werden die Begriffe »Diuretikum« und »diuretisch« zur allgemeinen Bezeichnung der diuretischen und/oder saluretischen Wirkung verwendet; im engeren Sinne bedeutet jedoch die Diurese eine gegenüber normal erhöhte Ausscheidung der Harnmenge, die Salurese dann eine solche der Elektrolyten, insbesondere von Natrium-, Kalium- und Chloridionen.

Diuretika haben eine breite therapeutische Verwendung. So eignen sie sich ausgezeichnet z. B. zur Behandlung von cardiovasculären Erkrankungen, wie der Stauungsinsuffizienz des Herzens. Der mit Hilfe der Diuretika bewirkte Wasser- und Elektrolytenverlust übt einen bemerkenswert günstigen Einfluß bei der medizinischen Behandlung von peripheren Ödemen, Lungenstauung (Dyspnoe, Orthopnoe, Husten), Ascites und Pleuraerguss aus. Diuretika ermöglichen ferner eine wirksame Behandlung von bei Nephose und bestimmten Nephritistypen auftretenden Ödemen. Die Verabreichung dieser Arzneistoffe hat in diesem Falle eine sofortige Ausscheidung zurückgehaltener Flüssigkeit und Elektrolyte und eine entsprechende Erleichterung für den Patienten zufolge. Diuretika eignen sich insbesondere für die Behandlung von Hypertonie und führen zu guten Heilerfolgen sowohl bei milden wie mittelschweren und schweren Formen dieser Erkrankung.

Obwohl Diuretika dank dieser günstigen therapeutischen Wirkung häufig lebensrettend sind, weisen die meisten von ihnen den Nachteil auf, daß sie in bezug auf Elektrolyte unspezifisch sind, d. h., daß sie neben der erwünschten Ausscheidung von Natriumionen in gleichem Maße auch die Ausscheidung beträchtlicher Mengen von Kaliumionen bewirken. Die dadurch verursachten Verluste an Kaliumionen führen zu Störungen des Herzrhythmus und Muskelschwäche und äußern sich durch ein Gefühl völliger körperlicher Erschöpfung. Diese unerwünschte Nebenwirkung, welche auf das gestörte Gleichgewicht von Elektrolyten infolge einer übermäßigen Kaliumausscheidung zurückzuführen ist, bedeutet insbesondere bei einer Dauertherapie, wie z. B. bei der Behandlung von kongestiver Herzinsuffizienz oder Hypertonie, eine schwerwiegende Beeinträchtigung der Heilwirkung herkömmlicher Diuretika. Deshalb wurden physiologische bzw. therapeutische Lösungen angestrebt, die von diesen Nebenerscheinungen frei sind und durch welche bei einem Warmblüter, vor allem beim Menschen, eine wirksame Diurese erzielt wird, die bei einer möglichst hohen Ausscheidung von Natriumionen jedoch eine relativ geringe Ausscheidung von Kaliumionen bewirkt.

Diese Zielsetzung erreicht man durch die Verabreichung der vorliegenden erfindungsgemäßen Arzneimittel, die dadurch gekennzeichnet sind, daß sie eine Aldosteron-antagonisierende Steroid-Komponente (Komponente A), bestehend aus mindesetns einer 19-oxygenierten Pregnanverbindung, und eine in bezug auf die Elektrolytausscheidung unspezifische diuretische Komponente (Komponente B) im Verhältnis von 4 : 1 bis 1 : 4 (bezogen auf die jeweilige mittlere effektive Dosis) gegebenenfalls zusammen mit pharmazeutischen Trägermaterialien oder Hilfsstoffen enthalten. Gegenstand der Erfindung sind ferner Arzneimittel, enthaltend eine therapeutisch wirksame Menge von jeder der beiden obengenannten Komponenten A und B in einer Dosierungsform.

Zwar sind bereits aus C. A., 54 (1960), 23 057 h sowie GB-A-1 041 534 Zusammensetzungen bekannt, die ein Diuretikum und einen Aldosteronantagonisten enthalten.

Ein besonderer Vorteil der obengenannten Aldosteron-antagonisierenden Steroid-Komponente A besteht im praktisch vollständigen Ausbleiben der üblichen Sexualspezifischen Nebenwirkungen, wie einer antiandrogenen Wirkung, was insbesondere für die Dauertherapie von großer Bedeutung ist. Als Beispiel des Anwendungsgebietes der vorliegenden Erfindung sei die Behandlung von Herzinsuffizienz, Rhythmusstörungen infolge Kaliummangel, Cor pulmonale, Lebercirrhose, Hypertonie sowie die Ascites-Behandlung erwähnt.

Die Aldosteron-antagonisierende Steroid-Komponente A besteht aus mindestens einer 19-oxygenierten Verbindung der Pregnan-Reihe der Formel

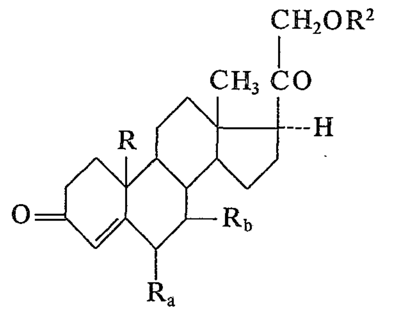

(I)

worin $R_a$ ein Wasserstoffatom und $R_b$ eine $\alpha$-orientierte $C_1-C_4$-Alkanoylthiogruppe bedeutet, oder $R_a$ und $R_b$ zusammen für eine Kohlenstoff-Kohlenstoff-Bindung stehen, R eine freie oder durch eine

gerades $C_1-C_4$-Alkyl verätherte oder durch ein $C_1-C_7$-Alkanoyl veresterte Hydroxylmethylgruppe darstellt und $R^2$ ein Wasserstoff oder den Acylrest Ac einer Carbonsäure mit 1–8 C-Atomen bedeutet, wie sie in der DE-A-2 823 239 beschrieben sind.

Diese Verbindungen zeichnen sich durch die gewünschten günstigen biologischen Eigenschaften aus, indem sie im Tierversuch eine starke Aldosteron-antagonistische Wirkung aufweisen und die durch Aldosteron hervorgerufene übermäßige Natrium-Retention und Kalium-Exkretion herabsetzen.

Als Komponente A sind unter den Aldosteron-antagonisierenden Steroiden der Formel I diejenigen bevorzugt, worin $R_a$ Wasserstoff, $R_b$ $\alpha$-Acetylthio, R Hydroxymethyl oder $C_1-C_7$-Alkanoyloxymethyl, insbesondere Acetoxymethyl, und $R^2$ Wasserstoff oder $C_1-C_8$-Alkanoyl, insbesondere Acetyl, bedeutet, vor allem 7$\alpha$-Acetylthio-19,21-dihydroxypregn-4-en-3,20-dion, sein 19-Acetat, 21-Acetat und 19,21-Diacetat.

Ganz besonders bevorzugt als Komponente A sind unter den Verbindungen der obigen Formel I diejenigen Aldosteron-antagonisierenden Steroide, die durch die Formel II

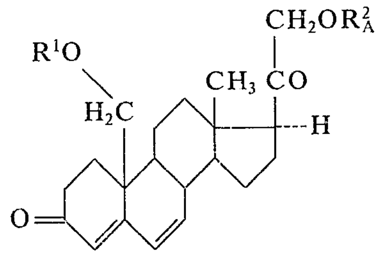

(II)

charakterisiert sind,
worin $R^1$ ein $C_1-C_7$-Alkanoylrest oder ein Wasserstoffatom und $R_A^2$ ein Acylrest Ac einer Carbonsäure mit 1–8 C-Atomen oder ein Wasserstoffatom ist, vor allem worin $R^1$ und $R_A^2$ je ein Wasserstoffatom bedeuten. Als ein Acylrest Ac ist ein linearer Alkanoylrest, vor allem der Acetylrest besonders bevorzugt. Spezifisch hervorzuheben sind 19,21-Dihydroxy-pregna-4,6-dien-3,20-dion, sein 19,21-Diacetat sowie 19-Acetat und 21-Acetat. Von den $C_1-C_7$-Alkenoylresten sind diejenigen mit nicht mehr als 4 Kohlenstoffatomen bevorzugt.

In den oben charakterisierten Formeln I und II leitet sich der Acylrest Ac von den in der Steroidchemie gebräuchlichen Carbonsäuren ab, z. B. von aliphatischen Monocarbonsäuren mit 1–8 C-Atomen, wie der Valerian-, Isovalerian-, Trimethylessig-, Hexan- 2,2-Dimethylbutter- und Heptansäure und insbesondere von geraden oder verzweigten Niederalkansäuren, wie Ameisen-, Propion-, Butter-, Isobutter- und vor allem Essigsäure. Man kann aber auch Säuren verwenden, welche ungesättigt und/oder in üblicher Weise substituiert sind, z. B. Phenyl- und Cyclohexylessigsäure, Phenoxyessigsäure, 4-Cyclopentylpropionsäure, Chloressigsäure und Trifluoressigsäure, Aminoessigsäure, $\alpha$- oder $\beta$-Oxypropionsäure sowie Benzoesäure.

Eine $C_1-C_4$-Alkanoylthiogruppe leitet sich von den entsprechenden $C_1-C_4$-Alkansäuren ab, vor allem ist sie die Acetylthiogruppe.

Ein $C_1-C_4$-Alkylrest ist ein solcher mit gerader Kohlenstoffkette, z. B. Äthyl, Propyl, Butyl und insbesondere Methyl. Bevorzugte $C_1-C_4$-Alkoxyreste entsprechen den genannten bevorzugten $C_1-C_4$-Alkylresten; besonders bevorzugt ist der Methoxyrest.

Als die in bezug auf Elektrolytausscheidung unspezifische diuretische Komponente B herkömmliche »klassische« Diuretika oder deren Gemische in Betracht, die sowohl durch renale als auch durch extrarenale Wirkung auf Gewebe die Diurese erhöhen, insbesondere Substanzen mit hemmender Wirkung auf die Rückresorption im Tubulus, wie Saluretika oder Äthacrinsäure und deren Analoge.

Insbesondere geeignet als die Elektrolyt-unspezifische Komponente B sind Benzothiadiazin-Derivate, wie Thiazide und Hydrothiazide, ferner Benzolsulfonamide, Phenoxyessigsäuren, Benzofuran-2-carbonsäuren und 2,3-Dihydro-benzofuran-2-carbonsäuren. Die Elektrolyt-unspezifische Komponente B kann aus einem einzelnen Wirkstoff oder einer zweckmäßigen Kombination mehrerer Wirkstoffe bestehen, wobei die Wirkstoffe auch mehreren der genannten Stoffgruppen angehören können.

Besonders geeignete Thiazide sind solche der Formel III,

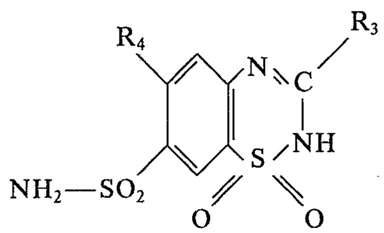

(III)

worin $R_3$ ein Wasserstoffatom oder ein Phenylniederalkylthioniederalkyl und $R_4$ ein Halogenatom oder die Trifluormethylgruppe sind.

Ein Phenylniederalkyl-thio-niederalkylrest $R_3$ ist insbesondere ein solcher, worin beide Niederalkylteile je höchstens 4 C-Atome und besonders 1 C-Atom aufweisen, und worin der Phenylteil unsubstituiert ist, wie der Benzylthiomethylrest.

Ein Halogenatom $R_4$ ist ein Bromatom, Jodatom, Fluoratom und insbesondere ein Chloratom.

Vor allem zu nennen sind von diesen Verbindungen der Formel III 6-Chlor-7-sulfamyl-1,2,4-benzothiadiazin-1,1-dioxyd, 6-Trifluormethyl-7-sulfamyl-1,2,4-benzothiadiazin-1,1-dioxyd und 2-Benzylthiomethyl-6-chlor-7-sulfamyl-1,2,4-benzothiadiazin-1,1-dioxyd.

Besonders geeignete Hydrothiazide sind solche der Formel IV,

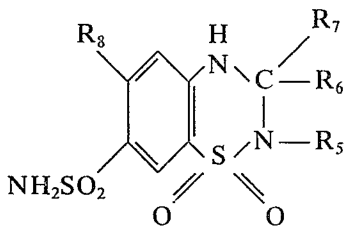

(IV)

worin $R_5$ Wasserstoff oder Niederalkyl ist, $R_6$ Wasserstoff, Niederalkyl, Niederalkenyl, Cycloalkyl, Cycloalkenyl, Cycloalkyl-niederalkyl, Aryl, Aryl-niederalkyl, Halogen-niederalkyl, Niederalkylthio-niederalkyl, Niederalkenylthio-niederalkyl, Halogen-niederalkylthio-niederalkyl, Phenylniederalkyl-thio-niederalkyl oder Heterocyclylniederalkyl ist, $R_7$ Wasserstoff ist oder zusammen mit $R_6$ für Niederalkylen steht, und $R_8$ Halogen oder Trifluormethyl ist.

Ein Niederalkylrest $R_5$ ist insbesondere ein solcher mit höchstens 4 C-Atomen, wie Äthyl, n-Propyl, i-Propyl, gerades oder verzweigtes, in beliebiger Stellung gebundenes Butyl, und vor allem Methyl.

Ein Niederalkylrest $R_6$ ist insbesondere ein solcher, wie für $R_5$ angegeben, und vor allem Methyl, Äthyl oder Isobutyl.

Ein Niederalkenylrest $R_6$ ist insbesondere ein solcher mit höchstens 4 C-Atomen, insbesondere Allyl.

Ein Cycloalkylrest $R_6$ hat 3—8, vorzugsweise 5—7 Ring-C-Atome und zählt insgesamt höchsetns 8, insbesondere nicht mehr als 7 C-Atome, wie Cyclopentyl und Cyclohexyl. Ein Cycloalkenylrest $R_6$ ist ein analoger Rest mit einer Doppelbindung, z. B. Cyclopentenyl, Cyclohexenyl oder Norbornenyl, insbesondere 5-Norbornen-2-yl.

Ein Cycloalkyl-niederalkylrest $R_6$ ist ein solcher, worin der Cycloalkylteil und der Niederalkylteil obige Bedeutung haben, vor allem Cyclopentylmethyl.

Ein Arylrest $R_6$ ist vorzugsweise monocyclisch, wie Phenyl, und kann durch Niederalkyl, wie Methyl, Niederalkoxy, wie Methoxy, Halogen, wie Chlor, oder Trifluormethyl substituiert sein.

Ein Aryl-niederalkylrest $R_6$ ist insbesondere ein solcher, worin sowohl der Aryl- wie der Niederalkylteil die obige Bedeutung haben, wobei der Arylteil insbesondere unsubstituiertes Phenyl ist, wie im Benzyl- oder 1-Phenyläthylrest.

Ein Halogen-niederalkylrest $R_6$ ist insbesondere ein Niederalkylrest der obigen Bedeutung, der ein, zwei oder drei Halogenatome trägt, wie insbesondere ein Halogenmethyl, z. B. Trifluormethyl, Chlormethyl, Dichlormethyl und Trichlormethyl.

Ein Niederalkylthio-niederalkylrest $R_6$ ist insbesondere ein solcher, worin die beiden Niederalkylteile die oben angegebenen bevorzugten Bedeutungen haben, und ist vor allem Methylthiomethyl und 2-(Methylthio)-äthyl.

Ein Niederalkenylthio-niederalkylrest $R_6$ enthält je einen der obengenannten Niederalkyl- bzw. Niederalkenylreste und ist z. B. Allylthiomethyl.

Ein Halogenniederalkylthio-niederalkylrest $R_6$ ist insbesondere einer den obengenannten Niederalkylthio-niederalkylresten $R_6$ analoger Rest, der ein, zwei oder drei Halogenatome trägt, wie 2,2,2-Trifluoräthylthio-methyl.

Ein Phenylniederalkylthio-niederalkylrest $R_6$ ist insbesondere ein solcher, worin die beiden Niederalkylteile obige Bedeutung haben, wie Benzylthio-methyl.

Ein Heterocyclylniederalkylrest $R_6$ besteht aus einem 3- bis 10gliedrigen Ring, insbesondere einem 5gliedrigen Ring, der ein oder mehr Heteroatome hat, wie einem Furyl- oder einem Pyrrolylring, und einem Niederalkylrest, wie einem der obengenannten, vor allem Methyl.

Ein von $R_6$ und $R_7$ zusammen gebildeter Niederalkylenrest hat höchstens 7, vorzugsweise nicht mehr als 6, und mindestens 2, insbesondere mindestens 4 C-Atomen in der Alkylenkette, wie 1,5-Pentylen und 3-Methyl-1,5-pentylen.

Ein Halogenatom $R_8$ ist Brom, Jod oder Fluor und insbesondere Chlor.

Besonders zu nennen sind von diesen Verbindungen der Formel IV

3-Äthyl-6-chlor-7-sulfamyl-3,4-dihydro-1,2,4-benzothiadiazin-1,1-dioxyd;
3-Trichlormethyl-6-chlor-7-sulfamyl-3,4-dihydro-1,2,4-benzothiadiazin-1,1-dioxyd;
3-Benzyl-6-trifluoromethyl-7-sulfamyl-3,4-dihydro-1,2,4-benzothiadiazin-1,1-dioxyd;
2-Methyl-3-(2,2,2-trifluoräthylthiomethyl)-6-chlor-7-sulfamyl-3,4-dihydro-1,2,4-benzothiadiazin-1,1-dioxid;
3-(2,2,2-Trifluoräthylthiomethyl)-6-chlor-7-sulfamyl-3,4-dihydro-1,2,4-benzothiadiazin-1,1-dioxyd;
3-(5-Norbornen-2-yl)-6-chlor-7-sulfamyl-3,4-dihydro-1,2,4-benzothiadiazin-1,1-dioxyd;
2-Methyl-3-chlormethyl-6-chlor-7-sulfamyl-3,4-dihydro-1,2,4-benzothiadiazin-1,1-dioxyd;
6-Chlor-7-sulfamyl-3,4-dihydro-1,2,4-benzothiadiazin-1,1 dioxyd;
3-Dichlormethyl-6-chlor-7-sulfamyl-3,4-dihydro-1,2,4-benzothiadiazin-1,1-dioxyd;
3-Cyclopentylmethyl-6-chlor-7-sulfamyl-3,4-dihydro-1,2,4-benzothiadiazin-1,1-dioxyd;
6-Trifluormethyl-7-sulfamyl-3,4-dihydro-1,2,4-benzothiadiazin-1,1-dioxyd und
3-Isobutyl-6-chlor-7-sulfamyl-3,4-dihydro-1,2,4-benzothiadiazin-1,1-dioxyd.

Besonders geeignete Benzolsulfonamide sind solche der Formel V

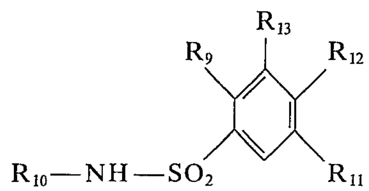

(V)

worin $R_9$ Halogen, Niederalkyl oder Trifluormethyl ist, $R_{10}$ Wasserstoff, Niederalkyl, Phenylniederalkyl, gegebenenfalls, z. B. durch Niederalkoxy oder Amino substituiertes Phenyl oder Pyrrolidinomethyl, Piperidinomethyl, Piperazinomethyl, Morpholinomethyl oder Thiomorpholinomethyl ist, $R_{11}$ Carboxyl, Carbamoyl, N-mono- oder N-di-substituiertes Carbamoyl, Sulfamoyl, N-mono- oder N-di-substituiertes Sulfamoyl, Niederalkylsulfonyl, oder ein Isoindolinyl ist, und kann gegebenenfalls mit dem Rest $R_{12}$ verbunden sein, $R_{12}$ Wasserstoff, Niederalkyl, Amino, mono- oder di-substituiertes Amino ist, und $R_{13}$ Wasserstoff oder Halogen ist; und insbesondere auch solche, worin $R_9$ Phenoxy, $R_{10}$ Wasserstoff, $R_{11}$ Carboxyl, $R_{12}$ Wasserstoff und $R_{13}$ Niederalkylamino, vor allem Butylamino, oder 3-(1-Pyrrolyl)-propyl ist.

Ein Niederalkylrest $R_9$, $R_{10}$ oder $R_{12}$ ist insbesondere einer der obengenannten, vor allem Methyl.

Ein Aminophenylrest $R_{10}$ ist insbesondere ein Monoaminophenylrest wie 4-Aminophenyl.

Ein N-monosubstituierter Carbamoylrest $R_{11}$ ist insbesondere ein N-Niederalkyl-carbamoylrest, worin Niederalkyl obige Bedeutung hat, wie der N-Methyl-carbamoylrest; er kann aber auch durch eine Aminogruppe N-substituiert sein, der Substituent ist dann insbesondere Diniederalkylamino, wie Dimethylamino, oder 1-Azacycloalkyl, wie Pyrrolidino, Piperidino oder 2,6-Dimethylpiperidino; der ganze Rest $R_{11}$ ist dann z. B. der N-(2,6-Dimethylpiperidino)-carbamoylrest.

Ein N-disubstituierter Carbamoylrest $R_{11}$ ist insbesondere ein N-Diniederalkyl-carbamoylrest, worin Niederalkyl obige Bedeutungen hat, z. B. der N,N-Dimethyl-carbamoylrest.

Ein N-monosubstituierter Sulfamoylrest $R_{11}$ ist insbesondere ein N-Niederalkylsulfamoylrest, worin Niederalkyl obige Bedeutung hat, wie N-Methyl-sulfamoyl, ferner ein N-Furfuryl- oder N-Tetrahydrofurfuryl-sulfamoylrest, wie N-Furfuryl-sulfamoyl, N-Tetrahydrofurfuryl-sulfamoyl, N-(2-Methyl-tetrahydrofurfuryl)-sulfamoyl und N-(2-Methyl-4-oxotetrahydrofurfuryl)-sulfamoyl.

Ein N-disubstituierter Sulfamoylrest $R_{11}$ ist insbesondere ein N,N-Diniederalkylsulfamoylrest, worin Niederalkyl obige Bedeutung hat, wie N,N-Dimethylsulfamoyl, ein (1-Azacycloalkyl)-sulfamoylrest, worin der 1-Azacycloalkylrest insbesondere obige Bedeutung hat, wie Piperidinosulfonyl, ein N-Niederalkyl-N-carboxyniederalkyl-sulfamoylrest, worin die Niederalkylteile obige Bedeutung haben, wie N-Methyl-N-carboxymethyl-sulfamoyl, ein N-Niederalkyl-N-furfuryl-sulfamoylrest, worin Niederalkyl obige Bedeutung hat, wie N-Methyl-N-furfurylsulfamoyl, oder ein N-Niederalkyl-N-tetrahydrofurfuryl-sulfamoylrest, worin Niederalkyl obige Bedeutungen hat, wie N-Methyl-N-(2-methyl-tetrahydrofurfuryl)-sulfamoyl und N-Methyl-N-(2-methyl-4-oxo-tetrahydrofurfuryl)-sulfamoyl.

Ein Niederalkylsulfonylrest $R_{11}$ ist insbesondere ein solcher, worin der Niederalkylteil obige Bedeutung hat, wie Methylsulfonyl, Äthylsulfonyl und n-Butylsulfonyl.

Ein Isoindolinylrest $R_{11}$ ist insbesondere ein 3-Isoindolinylrest mit einer 3-Hydroxygruppe und einer 1-Oxogruppe, wie 3-Hydroxy-1-oxo-isoindolinyl-(3).

Ein mit dem Rest $R_{12}$ verbundener Rest $R_{11}$ ist insbesondere ein Carbamoylrest $R_{11}$, der mit einer Aminogruppe $R_{12}$ oder mit einer Niederalkylgruppe $R_{12}$ verbunden ist, wie 1-Oxo-2-cyclohexyl-2-aza-propylen-(1,3) und 1-Oxo-2,4-bis-aza-3-äthyl-butylen-(1,4).

Eine monosubstituierte Aminogruppe $R_{12}$ ist insbesondere eine Niederalkylaminogruppe, worin Niederalkyl obige Bedeutung hat, wie Methylamino, oder Tetrahydrofurfurylamino oder insbesondere Furfurylamino oder Benzylamino.

Eine disubstituierte Aminogruppe $R_{12}$ ist insbesondere eine Diniederalkylaminogruppe, worin Niederalkyl obige Bedeutung hat, wie Dimethylamino, oder eine Di-(phenylniederalkyl)-aminogruppe, worin der Niederalkylteil obige Bedeutung hat, wie Dibenzylamino.

Ein Halogenatom $R_{13}$ ist Brom, Jod, Fluor oder insbesondere Chlor.

Von den Verbindungen der Formel V sind insbesondere solche hervorzuheben, worin $R_9$ Chlor ist, $R_{10}$

Aminophenyl, Methyl oder insbesondere Wasserstoff ist, $R_{11}$ Carboxyl, Carbamoyl, N-Methyl-carbamoyl, N-(2,6-Dimethyl-piperidino)-carbamoyl, 3-Hydroxy-1-oxo-isoindolinyl-(3) oder N-Methyl-N-(2-methyl-tetrahydrofurfuryl)-sulfamoyl ist oder zusammen mit $R_{12}$ für 1-Oxo-2-cyclohexyl-2-aza-propylen-(1,3) oder 1-Oxo-2,4-bis-aza-3-äthyl-butylen-(1,4) steht, $R_{12}$ Wasserstoff oder Furfurylamino ist, und $R_{13}$ Wasserstoff ist.

Von diesen Verbindungen der Formel V sind insbesondere zu nennen:

2-Chlor-5-N-methyl-sulfonamido-benzol-sulfonamid;
2-Chlor-5-N,N-dimethylsulfonamido-benzol-sulfonamid;
2-Chlor-5-piperidinosulphonyl-benzol-sulfonamid;
2-Chlor-5-(N-carboxymethyl-N-methyl)-sulfonamido-benzol-sulfonamid;
2-Chlor-5-(N-furfuryl-sulfonamido)-benzol-sulfonamid;
2-Chlor-5-(N-tetrahydrofurfuryl-sulfonamido)-benzol-sulfonamid;
2-Chlor-5-[N-methyl-N-(2-methyl-4-oxo-tetrahydrofurfuryl)-sulfonamido]-benzol-sulfonamid;
4,5-Dichlorbenzol-1,3-disulfonamid;
4-Chlor-6-methylbenzol-1,3-disulfonamid;
4-Chlor-6-aminobenzol-1,3-disulfonamid;
2-Chlor-5-methylsulphonyl-benzol-sulfonamid;
2-Chlor-5-äthylsulfphonylbenzol-sulfonamid;
2-Chlor-5-n-butylsulphonyl-benzol-sulfonamid;
2-Methyl-5-äthylsulphonylbenzol-sulfonamid;
2-Methyl-5-methylsulphonyl-benzol-sulfonamid;
2-Methyl-5-n-butylsulphonyl-benzol-sulfonamid;
2-Chlor-4-(N,N-dibenzylamino)-5-carboxyl-benzol-sulfonamid;
2-Furfurylamino-4-chlor-5-N-(p-aminophenyl)-sulphamoyl-benzoesäure;
2-Furfurylamino-4-chlor-5-N-(O-aminophenyl)-sulphamoylbenzoesäure und besonders
3-Sulfonamido-4-chlor-benzoesäure;
3-Sulfonamido-4-chlor-benzamid;
3-(N-methylsulphamoyl)-4-chlor-N-methylbenzamid;
1-Chlor-4-[N-methyl-N-(2-methyltetrahydrofurfuryl)-sulfamoyl]benzolsulfonamid;
1,3-Disulfamoyl-4-chlorbenzol;
2-Chlor-5-[3-hydroxy-1-oxoisoindolyl-(3)]-benzol-sulfonamid;
2-Äthyl-4-oxo-6-sulfamoyl-7-chlor-1,2,3,4-tetrahydro-chinazolin;
1-Oxo-2-cyclohexyl-5-chlor-6-sulfamoyl-1,2-dihydroisoindol;
2-Chlor-5-[N-(2,6-dimethylpiperidino)-carbamoyl]-benzolsulfonamid;
2-Chlor-4-furfurylamino-5-carboxyl-benzolsulfonamid und
2-Chlor-4-benzylamino-5-carboxyl-benzolsulfonamid.

Besonders geeignete Phenoxyessigsäuren sind solche der Formel VI

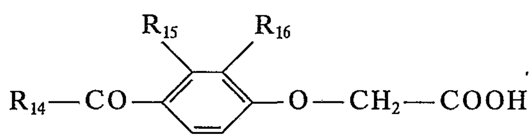

(VI)

worin $R_{14}$ Furyl, Thienyl, Niederalkyl-thienyl oder 1-Niederalkylvinyl ist, $R_{15}$ Halogen oder Niederalkyl ist und $R_{16}$ Wasserstoff, Halogen oder Niederalkyl ist oder worin $R_{15}$ und $R_{16}$ zusammen für den Rest eines anelierten Benzolrings, d. h. für 1,3-Butadienylen-1,4 stehen.

Das Niederalkyl im 1-Niederalkylvinyl hat vorzugsweise 2–7, vor allem 2–4 C-Atome, und ist vorzugsweise unverzweigt; es bildet somit z. B. 1-Propyl-, 1-Butyl- und insbesondere 1-Äthylvinyl.

Ein Halogenatom $R_{15}$ oder $R_{16}$ ist Brom, Jod oder Fluor und insbesondere Chlor, vor allem im Falle, wenn $R_{14}$ einer der obengenannten Heterocyclylreste ist.

Ein Niederalkylrest $R_{15}$ oder $R_{16}$ ist insbesondere ein solcher mit höchsetns 4 C-Atomen, insbesondere Methyl.

Zu Phenoxyessigsäuren sind auch Verbindungen der Formel VIA

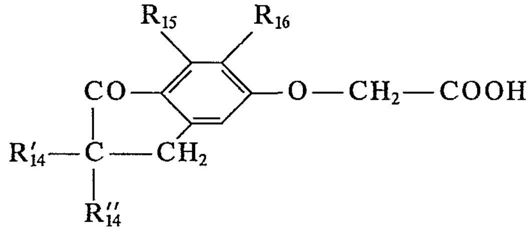

(VIA)

zu zählen, worin $R_{15}$ und $R_{16}$ die obengenannte Bedeutung haben, und insbesondere für Chlor oder Methyl stehen, $R_{14}'$ Niederalkyl mit höchstens 3 C-Atomen, insbesondere Methyl, und $R_{14}''$ Niederalkyl mit höchstens 3 C-Atomen, Phenyl, p-Chlorphenyl oder Thienyl bedeutet.

Als geeignete Verbindungen der Formel VI bzw. VIA sind insbesondere zu nennen:

a) [2,3-Dimethyl-4-(2-methylenbutyryl)-phenoxy]-essigsäure,
[2-Methyl-3-chlor-4-(2-methylen-butyryl)-phenoxy]-essigsäure,
[4-(2-Methylenbutyryl)-1-naphthoxy]-essigsäure und vor allem
[2,3-Dichlor-4-(2-methylen-butyryl)-phenoxy]-essigsäure;

b) 4-Thenoyl-2,3-dichlorphenoxyessigsäure,
4-(5-Methylthenoyl)-2,3-dichlorphenoxyessigsäure und
4-Furoyl-2,3-dichlorphenoxyessigsäure und

c) (1-Oxo-2-methyl-2-phenyl-6,7-dichlor-5-indanyloxy)-essigsäure
(insbesondere als Razemat oder die laevo-Form), oder auch
[1-Oxo-2-(4-chlorphenyl)-6,7-dichlor-5-indanyloxy]-essigsäure und
[1-Oxo-2-(2-thienyl)-6,7-dichlor-5-indanyloxy]-essigsäure.

Besonders geeignete Benzofuran-2-carbonsäuren sind solche der Formel VII

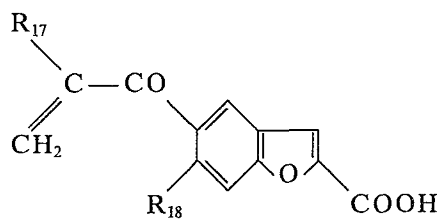

(VII)

worin $R_{17}$ Niederalkyl und $R_{18}$ Niederalkyl oder Niederalkoxy ist.

Ein Niederalkylrest $R_{17}$ ist insbesondere ein solcher mit 2—7, vor allem 2—4 C-Atomen, wie ein obengenannter, und vorzugsweise ein unverzweigter derartiger Rest, wie Äthyl.

Ein Niederalkylrest $R_{18}$ ist insbesondere ein solcher mit höchstens 4 C-Atomen und insbesondere Methyl.

Ein Niederalkoxyrest $R_{18}$ ist insbesondere ein solcher, worin der Alkylteil die unmittelbar vorangehende Bedeutung hat, wie Methoxy.

Als geeignete Verbindungen der Formel VII sind insbesondere zu nennen: 5-(2-Methylen-butyryl)-6-methyl-benzofuran-2-carbonsäure, 5-(2-Methylen-butyryl)-6-methoxy-benzofuran-2-carbonsäure und 5-(2-methylen-propionyl)-6-methylbenzofuran-2-carbonsäure.

Besonders geeignete 2,3-Dihydro-benzofuran-2-carbonsäuren sind solche der Formel VIII,

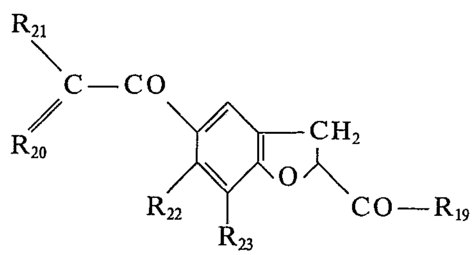

(VIII)

worin $R_{19}$ Hydroxyl, Alkoxy, Cycloalkoxy oder Aralkoxy ist, $R_{20}$ für zwei Wasserstoffatome oder Niederalkyliden steht, $R_{21}$ Niederalkyl, $R_{22}$ Wasserstoff, Halogen, Niederalkyl oder Niederalkoxy und $R_{23}$ Wasserstoff oder Niederalkyl ist.

Ein Alkoxyrest $R_{19}$ ist ein solcher mit 1—18, insbesondere 1—12 C-Atomen, wie Methoxy, Äthoxy, n-Butoxy, 2-Hexyloxy und n-Decyloxy.

Ein Cycloalkoxyrest $R_{19}$ ist ein solcher mit 3—8, insbesondere 5—7 Ring-C-Atomen und vorzugsweise mit höchstens 10, vor allem bis zu 8 C-Atomen, wie Cyclopentyloxy und Cyclohexyloxy.

Ein Aralkoxyrest $R_{19}$ ist insbesondere Phenylniederalkoxy, worin der Niederalkylteil höchstens 4 C-Atome aufweist und obige Bedeutung hat, vor allem Benzyloxy.

Ein Niederalkylidenrest $R_{20}$ ist insbesondere ein solcher mit höchstens 4 C-Atomen, vor allem Methylen und Äthyliden.

Ein Niederalkylrest $R_{21}$ ist insbesondere ein solcher mit höchstens 4 C-Atomen, und ist vorzugsweise geradkettig, wie Methyl, n-Propyl, n-Butyl und insbesondere Äthyl.

Ein Halogenatom $R_{22}$ kann Brom oder Jod sein; vorzugsweise ist es Fluor oder vor allem Chlor.

Ein Niederalkylrest $R_{22}$ oder $R_{23}$ ist insbesondere ein solcher mit höchstens 4 C-Atomen, wie ein obengenannter, vor allem Methyl.

Ein Niederalkoxyrest $R_{22}$ ist insbesondere ein solcher mit höchstens 4 C-Atomen, wie ein

obengenannter, vor allem Methoxy.

Von den Verbindungen der Formel VIII sind solche bevorzugt, worin $R_{19}$ Hydroxyl, $R_{20}$ Methylen oder Äthyliden, $R_{21}$ geradkettiges Alkyl mit 1—4 C-Atomen, $R_{22}$ Methyl, Methoxy, Chlor oder Fluor und $R_{23}$ Wasserstoff oder Methyl ist.

Von den Verbindungen der Formel VIII seien hervorgehoben

5-(2-Methylen-butyryl)-6-methyl-2,3-dihydrobenzofuran-2-carbonsäure;
5-(2-Methylen-butyryl)-6-fluor-2,3-dihydro-benzofuran-2-carbonsäure;
5-(2-Methylenbutyryl)-6-chlor-2,3-dihydro-benzofuran-2-carbonsäure;
5-(2-Methylenpropionyl)-6-methyl-2,3-dihydro-benzofuran-2-carbonsäure;
5-(2-Methylen-hexanoyl)-6-methyl-2,3-dihydro-benzofuran-2-carbonsäure;
5-(2-Methylen-valeryl)-6-methyl-2,3-dihydrobenzofuran-2-carbonsäure;
5-(2-Methylenpropionyl)-6-methyl-2,3-dihydro-benzofuran-2-carbonsäure;
5-(2-Äthyliden-butyryl)-6-methyl-2,3-dihydro-benzofuran-2-carbonsäure;
5-(2-Methylenbutyryl)-6-methyl-2,3-dihydro-benzofuran-2-carbonsäuremethylester;
5-(2-Methylenbutyryl)-6-methyl-2,3-dihydrobenzofuran-2-carbonsäureäthylester;
5-(2-Methylenbutyryl)-6-methyl-2,3-dihydro-benzofuran-2-carbonsäure-n-butylester;
5-(2-Methylenbutyryl)-6-methyl-2,3-dihydro-benzofuran-2-carbonsäure-2-hexylester;
5-(2-Methylenbutyryl)-6-methyl-2,3-dihydro-benzofuran-2-carbonsäure-n-decylester;
5-(2-Methylenbutyryl)-6-methyl-2,3-dihydro-benzofuran-2-carbonsäure-cyclopentylester;
5-(2-Methylenbutyryl)-6-methyl)-2,3-dihydrobenzofuran-2-carbonsäure-cyclohexylester;
5-(2-Methylenbutyryl)-6-methyl-2,3-dihydro-benzofuran-2-carbonsäure-benzylester;
5-(2-Methylenbutyryl)-7-methyl-2,3-dihydro-benzofuran-2-carbonsäure-methylester;
5-(2-Methylenbutyryl)-6-chlor-7-methyl-2,3-dihydro-benzofuran-2-carbonsäure-methylester;
5-(2-Methylenpropionyl)-6-methyl-2,3-dihydro-benzofuran-2-carbonsäuremethylester;
5-(2-Methylen-valeryl)-6-methyl-2,3-dihydro-benzofuran-2-carbonsäure-methylester;
5-(2-Methylen-3-methyl-butyryl)-6-methyl-2,3-dihydro-benzofuran-2-carbonsäure-methylester;
und
5-(2-Methylenbutyryl)-6-fluor-2,3-dihydro-benzofuran-2-carbonsäure-methylester,
und besonders
5-(2-Methylenbutyryl)-6,7-dimethyl-2,3-dihydro-benzofuran-2-carbonsäure;
5-(2-Methylen-3-methyl-butyryl)-6-methyl-2,3-dihydro-benzofuran-2-carbonsäure;
5-(2-Methylenbutyryl)-6-chlor-7-methyl-2,3-dihydro-benzofuran-2-carbonsäure.

Die genannten Diuretika der Formeln III bis VIII können je nach Anzahl ihrer asymmetrischen Kohlenstoffatome in Form von Isomerengemischen, reinen Isomeren (Racematen) oder optischen Antipoden vorliegen. Vorzugsweise verwendet man sie jeweils in Form des besser wirksamen bzw. weniger toxischen Isomeren bzw. Antipoden.

Die genannten Diuretika der Formeln III—V mit basischen Gruppen können ferner in freier Form oder in Form ihrer nichttoxischen Salze vorliegen. Als solche Salze kommen insbesondere Salze mit organischen oder anorganischen Säuren in Betracht, wie z. B. Halogenwasserstoffsäuren, Schwefelsäuren, Phosphorsäuren, Salpetersäure, Perchlorsäure, aliphatische, alicyclische, aromatische oder heterocyclische Carbon- oder Sulfonsäuren, wie Ameisen-, Essig-, Propion-, Bernstein-, Glykol-, Milch-, Äpfel-, Wein-, Zitronen-, Ascorbin-, Malein-, Hydroxymalein- oder Brenztraubensäure; Phenylessig-, Benzoe-, p-Aminobenzoe-, Anthranil-, p-Hydroxybenzoe-, Salicyl- oder p-Aminosalicylsäure, Methansulfon-, Äthansulfon-, Hydroxyäthansulfon-, Äthylensulfonsäure; Halogenbenzolsulfon-, Toluolsulfon-, Naphthalinsulfonsäure oder Sulfanilsäure; Cyclohexyl-sulfaminsäure, Methionin, Tryptophan, Lysin oder Arginin.

Die genannten Diuretika mit sauren Gruppen der Formeln VI bis VIII können ebenfalls in freier Form oder in Form ihrer nichttoxischen Salze vorliegen. Als solche Salze kommen insbesondere Salze mit Basen in Betracht, wie sie eingangs bei den Verbindungen der Formel I genannt wurden. Aluminiumsalze, z. B. Salze aus zwei Mol Säure und einem Mol Aluminiumhydroxyd, sind ebenfalls geeignet, insbesondere wegen ihrer langsameren Resorption, Geruchlosigkeit und der geringen gastrointestinalen Störungen.

Die Erfindung betrifft auch die Herstellung der erfindungsgemäßen Zusammensetzungen und Arzneimittel, ferner die Kombination der Wirkstoffe A und B, und zwar sowohl in Form der genannten Arzneimittel wie auch durch eine kombinierte aber getrennte Verabreichung beider Wirkstoffe, zur Anwendung bei der Behandlung von krankhaften Zuständen, die mit gestörter Ausscheidung von Harn bzw. Harnbestandteilen, wie insbesondere von Wasser und Elektrolyten, einhergehen.

Besonders wertvoll sind pharmazeutische Zusammensetzungen und Arzneimittel, enthaltend die oben als bevorzugt hervorgehobenen Wirkstoffe der Formeln I—VIII, z. B. als die Aldosteron-antagonisierende Komponente A das 19,21-Dihydroxypregna-4,6-dien-3,20-dion, sein 19,21-Diacetat, 19-Monoacetat oder 21-Monoacetat und als die in bezug auf Elektrolyte unspezifisch diuretische Komponente B

1-Oxo-3-(3-sulfamyl-4-chlor-phenyl)-3-hydroxyisoindolin,
6-Chloro-7-sulfamyl-3,4-dihydro-1,2,4-benzothiadiazin-1,1-dioxyd,
3-Cyclopentylmethyl-6-chloro-7-sulfamyl-3,4-dihydro-1,2,4-benzothiadiazin-1,1-dioxyd,
4-(2-Methylenbutyryl)-2,3-dichlor-phenoxyessigsäure,
4-Thenoyl-2,3-dichlor-phenoxyessigsäure,
[1-Oxo-2-methyl-2-phenyl-6,7-dichlor-5-indanyloxy)-essigsäure,
2-Chlor-4-furfurylamino-5-carboxybenzolsulfonamid,
2-Phenoxy-3-butylamino-5-carboxybenzolsulfonamid oder
2-Phenoxy-3-[3-(1-pyrrolyl)-propyl]-5-carboxybenzolsulfonamid.

In den erfindungsgemäßen pharmazeutischen Zusammensetzungen und Arzneimitteln beträgt das Verhältnis der Komponente A zur Komponente B, bezogen auf die jeweilige mittlere effektive Dosis, 4 : 1 bis 1 : 4, vorzugsweise 3 : 2 bis 2 : 3. Da die mittlere effektive Dosis jeder spezifischen Komponente ein bekannter, oder durch bekannte pharmakologische Testmethoden einfach feststellbarer Wert ist, ist es dem Fachmann leicht möglich, innerhalb der obengenannten Grenzen ein geeignetes Verhältnis beider Komponenten jedem Patienten gemäß seinem spezifischen Leiden, allgemeinen Gesundheitszustand, individueller Ansprechbarkeit und Alter, sowie auch der Species, zu verordnen. Auch die Größe der Dosierungseinheiten der erfindungsgemäßen Arzneimittel hängt natürlich in erster Linie von der Wirksamkeit der jeweiligen Komponenten A bzw. B, wie sie vorzugsweise durch die Tagesdosis ausgedrückt ist, ab. Der Ausdruck »Dosierungseinheit« ist in dieser Beziehung angewendet zur Bezeichnung von einzelnen abgetrennten Portionen einheitlicher Zusammensetzung, welche für die medizinische Verabreichung geeignet sind und je einzeln eine spezifische Menge der erfindungsgemäßen Wirkstoffe enthalten, die 0,05 bis 2, vorzugsweise 0,15 bis 1 Tagesdosis entspricht.

So können beispielsweise die obengenannten besonders bevorzugten Präparate pro Dosiseinheit 15 bis 150 mg, insbesondere 20 bis 100 mg 19,21-Dihydroxypregna-4,6-dien-3,20-dion oder eines der genannten Acetate als Komponente A enthalten. Der Gehalt an Komponente B beträgt beispielsweise 10—100 mg, insbesondere 25—50 mg 2-Chlor-5-[3-hydroxy-1-oxo-isoindolyl-(3)]-benzol-sulfonamid oder 4-(2-Methylenbutyryl)-2,3-dichlorphenoxyessigsäure, 5—50 mg, insbesondere 12—25 mg 6-Chloro-7-sulfamyl-3,4-dihydro-1,2,4-benzothiadiazin-1,1-dioxyd oder 2-Chlor-4-furfurylamino-5-carboxybenzolsulfonamid, 2—20 mg, insbesondere 5—10 mg 2-Phenoxy-3-[3-(1-Pyrrolyl)-propyl]-5-carboxybenzolsulfonamid, 0,1—1,0 mg, insbesondere 0,25—0,5 mg 3-Cyclopentylmethyl-6-chloro-7-sulfamyl-3,4-dihydro-1,2,4-benzothiadiazin-1,1-dioxyd oder 2-Phenoxy-3-butylamino-5-carboxybenzolsulfonamid, 100—400 mg, insbesondere 200 mg 4-Thenoyl-2,3-dichlor-phenoxyessigsäure und 5—25 mg, insbesondere 10 mg racemische (1-Oxo-2-methyl-2-phenyl-6,7-dichlor-5-indanyloxy)-essigsäure, oder eine halbe Menge der laevo-Form dieser Säure.

Für die Ödembehandlung in einem mittelschweren Fall werden beispielsweise 1—3 Dosiseinheiten täglich genommen, die die Wirkstoffe in Gewichtsmengen enthalten, welche an der höheren Grenze der obenerwähnten besonders bevorzugten Dosierung liegen; ein mittelschwerer Fall der essentiellen Hypertonie wird z. B. mit 1—3 Dosiseinheiten behandelt, deren Wirkstoffgehalt an der unteren besonders bevorzugten Mengengrenze liegt.

Unter dem Begriff »Wirkstoff« ist vor- und nachstehend insbesondere eine Verbindung der Formeln I bis VIII gemeint, wie sie im Zusammenhang mit den erfindungsgemäßen pharmazeutischen Zusammensetzungen und Arzneimitteln durch die allgemeinen und insbesondere spezifischen Bedeutungen definiert ist. Der Begriff »pharmazeutische Zusammensetzung« bezieht sich auf Gemische von Wirkstoffen beider Typen, gegebenenfalls noch zusammen mit geeigneten Träger- bzw. Hilfsstoffen in einem gewünschten, zahlenmäßig festgesetzten Verhältnis, die nach den herkömmlichen pharmazeutischen Verfahren und Methoden zubereitet werden. Der Ausdruck »Arzneimittel« ist angewendet zur Bezeichnung von einzelnen abgetrennten Portionen einheitlicher Zusammensetzung, welche für die medizinische Verabreichung geeignet sind.

Wie oben bereits gesagt wurde, betrifft die Erfindung insbesondere Arzneimittel, vor allem Arzneimittel in Form von Dosierungseinheiten, und vorzugsweise in fester Form, enthaltend mindestens eine 19-oxygenierte Pregnan-Verbindung der Formel I als Aldosteron-antagonisierende Komponente A und mindestens ein in bezug auf Elektrolyte unspezifisches Diuretikum als Komponente B, gegebenenfalls zusammen mit mindestens einem pharmazeutischen Träger- oder Hilfsstoff. Die Erfindung betrifft insbesondere Arzneimittel in Form von Tabletten (einschließlich Lutschtabletten, Granulen und Pastillen), Dragées, Kapseln, Pillen oder Suppositorien, enthaltend die beiden Komponenten A und B ohne Beimischung oder im Gemisch mit einem oder mehreren Trägerstoffen.

Die erfindungsgemäßen pharmazeutischen Zusammensetzungen enthalten vorzugsweise 0,1 bis 99,5, insbesondere von 1 bis 90 Gewichts-% des Wirkstoff-Gemisches.

Die erfindungsgemäßen pharmazeutischen Zusammensetzungen und Arzneimittel sind insbesondere zur enteralen, vor allem oralen oder rektalen, Applikationen bestimmt. Vorzugsweise handelt es sich um Tabletten, Dragées oder in zweiter Linie um Suppositorien oder Kapseln, die jeweils neben den erfindungsgemäß zu verwendenden Wirkstoffen für die jeweilige Applikationsform übliche Hilfs- und

Trägerstoffe enthalten, die die Verarbeitung der Wirkstoffe in die gewünschten Formen erleichtern und eine gesteuerte Freisetzung der Wirkstoffe ermöglichen. Die neuen Arzneimittel können dabei auch als Punkttabletten oder Punktdragées formuliert werden. Diese bestehen im Prinzip aus einem die eine Wirkstoffkomponente enthaltenden Tabletten- bzw. Dragéekern, einem die andere Wirkstoffkomponente enthaltenden sogenannten Punkt, der auf den Kern aufgepreßt wird, und erforderlichenfalls üblichen Trenn-, Deck- und/oder Schutzschichten.

Der Kern ist vorzugsweise aus dem Wirkstoff bzw. einem diesen enthaltenden Wirkstoff-Hilfsstoffgemisch und gegebenenfalls Hilfs-, Träger- und/oder Hüllstoffen zusammengesetzt. Ein Wirkstoff-Hilfsstoff-Gemisch ist z. B. ein entsprechendes Granulat, das sich zur Verarbeitung zu Tabletten, Dragées oder Kapseln eignet. Ein solches Granulat enthält außer dem Wirkstoff u. a. Verdünnungs- oder Füllstoffe, wie Zucker, z. B. Lactose oder Saccharose, oder Zuckeralkohole, z. B. Mannit oder Sorbit, Cellulosepräparate und/oder Calciumphosphate, z. B. Tricalciumphosphat oder Calciumhydrogenphosphat, ferner Fließreguliermittel, wie Talk oder kolloidale Kieselsäure, Gleit-, Schmier- bzw. Antiklebemittel, wie Stearinsäure oder Salze davon, z. B. Magnesium- oder Calciumstearat, und/oder Polyäthylenglykol, ferner Glyceride, wie hydriertes Baumwollsamen- oder hydriertes Rizinusöl. Ein solches Granzlat kann in an sich bekannter Weise, gegebenenfalls unter Verwendung von Befeuchtungsmitteln, wie Wasser oder organischen Lösungsmitteln, z. B. Äthanol, oder Gemischen davon hergestellt werden.

Die pharmazeutischen Präparate der vorliegenden Erfindung werden in an sich bekannter Weise, z. B. mittels konventioneller Misch-, Granulier-, Dragier-, Lösungs- oder Lyophilisierungsverfahren hergestellt. So kann man Tabletten bzw. Dragée-Kerne zur oralen Anwendung erhalten, indem man die Wirkstoffe mit festen Trägerstoffen kombiniert, ein erhaltenes Gemisch gegebenenfalls granuliert, und das Gemisch bzw. Granulat, gewünschtenfalls oder nötigenfalls nach Zugabe von geeigneten Hilfsstoffen, zu Tabletten oder Dragée-Kernen verarbeitet.

Als Trägerstoffe kommen beispielsweise in Betracht: Zucker, z. B. Lactose, Saccharose, Mannit oder Sorbit, übliche Cellulosepräparationen, Calciumphosphate, z. B. Tricalcium- oder Calciumhydrogenphosphat, Bindemittel, wie Stärkekleister, z. B. auf Basis von Mais-, Weizen-, Reis- oder Kartoffelstärke, Gelatine, Traganth, Methylcellulose, Natriumcarboxycellulose und/oder Polyvinylpyrrolidon, ferner Carboxymethylstärke, quervernetztes Polyvinylpyrrolidon, Agar, Alginsäure bzw. ein Alginat, z. B. Natriumalginat, und/oder, wenn erwünscht, Sprengmittel, wie die obengenannten Stärken. Hilfsmittel sind in erster Linie Fließregulier- und Schmiermittel, z. B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Überzügen versehen, wobei man u. a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Durch Einarbeiten eines oder mehrerer Wirkstoffe in ein geeignetes Trägermaterial, das eine langsame Abgabe eines oder mehreren Wirkstoffe bewirkt, kann die Wirkungsdauer einer oder mehrerer an sich kurz wirksamen Komponenten verlängert bzw. einander angepaßt werden. Dies ist insbesondere der Fall, wenn eine der beiden Komponenten, z. B. die Komponente A, eine deutlich kürzere Wirkungsdauer gegenüber der anderen Komponente hat, Dann wird die kürzer wirksame Komponente in einen Retardkern, die länger wirksame in die umhüllende Schicht eingearbeitet. Den Tabletten oder Dragée-Überzügen können Farbstoffe oder Pigmente, z. B. zur Identifizierung oder zur Kennzeichnung verschiedener Kombinationen von Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können die Wirkstoffe in Form eines Granulats, z. B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In wechen Kapseln sind die Wirkstoffe vorzugsweise in geeigneten Flüssigkeiten, wie fetten Ölen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z. B. Suppositorien in Betracht, welche aus einer Kombination der Wirkstoffe mit einer Suppositoriengrundmasse bestehen. Als Suppositoriengrundmasse eignen sich z. B. natürliche oder synthetische Triglyceride, Paraffinkohlenwasserstoffe, Polyäthylenglykole oder höhere Alkanole. Ferner können auch Gelatine-Rektalkapseln verwendet werden, die aus einer Kombination der Wirkstoffe mit einer Grundmasse bestehen; als Grundmassenstoffe kommen z. B. flüssige Triglyceride, Polyäthylenglykole oder Paraffinkohlenwasserstoffe in Frage.

Wie oben ausgeführt, werden die erfindungsgemäßen enteral, vorzugsweise oral, verabreichbaren pharmazeutischen Präparate in an sich bekannter Weise hergestellt; die Herstellung wird in den nachfolgenden Beispielen näher erläutert.

Beispiel 1

Tabletten, enthaltend ca. 100 mg Komponente A und ca. 25 mg Komponente B, werden folgendermaßen hergestellt:

Zusammensetzung einer Tablette:

| | |
|---|---|
| Komponente A, mikronisiert | 100,0 mg |
| Komponente B, mikronisert | 25,0 mg |
| Maisstärke | 50,0 mg |
| Kieselsäure, kolloidal | 5,0 mg |
| Gelatine | 5,0 mg |
| Cellulose, mikrokristallin | 75,0 mg |
| Natriumcarboxymethylstärke | 20,0 mg |
| Magnesiumstearat | 1,5 mg |
| | 381,5 mg |

Herstellung von 100 000 Tabletten

10 kg Komponente A, mikronisiert, und 2,5 kg Komponente B, mikronisiert, 5,0 kg Maisstärke werden mit 0,5 kg kolloidaler Kieselsäure gemischt und mit einer Lösung von 0,5 kg Gelatine in 5,0 kg destilliertem Wasser (30° C) zu einer feuchten Masse verarbeitet. Diese wird durch ein Sieb von 3 mm Maschenweite getrieben und bei 45° C getrocknet (Wirbelschichttrockner). Das trockene Granulat wird durch ein Sieb von 0,8 mm Maschenweite gedrückt, mit einer vom voraus gesiebten Mischung von 7,5 kg mikrokristalliner Cellulose und 2,0 kg Natriumcarboxymethylstärke sowie 0,15 kg Magnesiumstearat gemischt und zu Tabletten von 381,5 mg Gewicht verpreßt.

Als Komponente A wird 19,21-Dihydroxypregna-4,6-dien-3,20-dion-19,21-diacetat, als Komponente B 6-Chlor-7-sulfamyl-3,4-dihydro-1,2,4-benzothiadiazin-1,1-dioxyd verwendet.

In analoger Weise kann man auch in den entsprechenden Mengen die folgenden Wirkstoffe einsetzen:

Als Komponente A:
19,21-Dihydroxypregna-4,6-dien-3,20-dion, sein 19-Acetat oder sein 21-Acetat (jeweils 100 mg);
als Komponente B:
2-Chlor-5-(3-hydroxy-1-oxo-isoindolyl-3)-benzolsulfonamid (50 mg),
4-(2-Methylenbutyryl)-2,3-dichlorphenoxy-essigsäure (50 mg),
(1-Oxo-2-methyl-2-phenyl-6,7-dichlor-indanyl-5-oxy)-essigsäure (als Razemat 20 mg, als die laevo-Form 10 mg) oder
3-Cyclopentylmethyl-6-chlor-7-sulfamyl-3,4-dihydro-1,2,4-benzothiadiazin-1,1-dioxid (0,5 mg).

Beispiel 2

Dragées, enthaltend ca. 100 mg Komponente A und 20 mg Komponente B werden folgendermaßen hergestellt:

Zusammensetzung eines Dragéekerns:

| | |
|---|---|
| Komponente A, mikronisiert | 100,0 mg |
| Komponente B, mikronisiert | 20,0 mg |
| Maisstärke | 90,0 mg |
| Tricalciumphosphat | 90,0 mg |
| Polyvinylpyrrolidon, K 25 | 15,0 mg |
| Magnesiumstearat | 2,0 mg |
| Natriumcarboxymethylstärke | 33,0 mg |
| | 350,0 mg |

Herstellung von 50 000 Dragéekernen

Die Mischung von 5 kg Komponente A, mikronisiert, 1,0 kg Komponente B, mikronisiert, 4,5 kg Maisstärke und 4,5 kg Tricalciumphosphat wird mit einer Lösung von 0,75 kg Polyvinylpyrrolidon K 25 in 5 kg destilliertem Wasser im Wirbelschichtverfahren granuliert. Dem bei 45° C getrockneten und durch ein Sieb von 1 mm Maschenweite gedrückten Granulat werden 0,1 kg Magnesiumstearat und 1,65 kg Natriumcarboxymethylstärke zugemischt und zu gewölbten Tabletten à 350 mg verpreßt.

Herstellung von 6,6 kg Zuckerdragées

6 kg der Dragéekerne werden in einem Dragierkessel von 45 cm Durchmesser mit einem Zuckersirup (2 Teile Zucker und 1 Gewichtsteil destilliertes Wasser), in dem 1,5% Polyvinylpyrrolidon K 25 und 1% Polyäthylenglykol 6000 gelöst sind sowie 20% Talk suspendiert ist, bis zu einem Gewicht von 410 mg portionsweise überzogen, dazwischen wird mit Warmluft von ca. 60° getrocknet. Anschließend wird Zuckersirup (2 Teile Zucker und 1 Teil Wasser) bis zum Endgewicht von 450 mg portionsweise aufgetragen. Die Dragées werden schließlich mit einer Lösung von 2% Carnaubawachs in Trichloräthylen geglänzt.

Als Komponente A wird 19,21-Dihydroxypregna-4,6-dien-3,20-dion-19,21-diacetat, als Komponente B 2-Chlor-4-furfurylamino-5-carboxybenzolsulfonamid verwendet.

In analoger Weise kann man auch in entsprechenden Mengen die folgenden Wirkstoffe einsetzen:

Als Komponente A:
19,21-Dihydroxypregna-4,6-dien-3,20-dion, sein 19-Acetat oder sein 21-Acetat (jeweils 100 mg);
als Komponente B:
2-Chlor-5-(3-hydroxy-1-oxo-isoindolyl-3)-benzolsulfonamid oder
4-(2-Methylenbutyryl)-2,3-dichlorphenoxyessigsäure (je 50 mg),
6-Chlor-7-sulfamyl-3,4-dihydro-1,2,4-benzothiadiazin-1,1-dioxid (25 mg) oder
2-Phenoxy-3-butylamino-5-carboxybenzolsulfonamid (0,5 mg).

Beispiel 3

Weichgelatinekapseln, enthaltend 50 mg Komponente A und 12,5 mg Komponente B werden folgendermaßen erhalten:

Zusammensetzung einer Weichgelatinekapsel:

| | |
|---|---|
| Komponente A, mikronisiert | 50,0 mg |
| Komponente B, mikronisiert | 12,5 mg |
| Sojalecithin | 1,5 mg |
| Bienenwachs | 2,5 mg |
| Pflanzenöl | 100,0 mg |
| Pflanzenöl, partiell hydriert | 54,0 mg |
| | 220,5 mg |

Herstellung von 100 000 Weichgelatinekapseln

6,25 kg eines gleichförmigen Gemisches der Komponenten A und B im Gewichtsverhältnis 4 : 1, mikronisiert, werden in einer durch Schmelzen hergestellten Mischung von 0,15 kg Sojalecithin, 0,25 kg Bienenwachs, 5,4 kg partiell hydriertem Pflanzenöl und 10 kg Pflanzenöl suspendiert und nach dem Stanzverfahren in Gelatinekapseln gebracht. Die Gelatinehülle besteht aus ca. 71% Gelatine, ca. 28% Glycerin (85%) und ca. 1% Titandioxid sowie 0,3% p-Hydroxybenzoesäurepropylester. Die Kapselgröße beträgt 4 minims (Form oblong).

Als Komponente A wird 19,21-Dihydroxypregna-4,6-dien-3,20-dion, als Komponente B 6-Chlor-7-Sulfamyl-3,4-dihydro-1,2,4-benzothiadiazin-1,1-dioxid verwendet.

In analoger Weise kann man auch in den entsprechenden Mengen die folgenden Wirkstoffe einsetzen:

Als Komponente A:
19,21-Dihydroxypregna-4,6-dien-3,20-dion-19-acetat, -21-acetat oder -19,21-diacetat (jeweils 50 mg);
als Komponente B:
3-Cyclopentylmethyl-6-chlor-7-sulfamyl-3,4-dihydro-1,2,4-benzothiadiazin-1,1-dioxid (0,25 mg),
4-Thienyl-2,3-dichlorphenoxyessigsäure (125 mg),
2-Chlor-5-(3-hydroxy-1-oxo-isoindolyl-3)-benzolsulfonamid (25 mg) oder
2-Chlor-4-furfurylamino-5-carboxybenzolsulfonamid (15 mg).

Beispiel 4

Filmdragées, enthaltend 100 mg Komponente A und 10 mg Komponente B werden folgendermaßen hergestellt:

Zusammensetzung eines Filmdragéekerns:

| | |
|---|---|
| Komponente A, mikronisiert | 100,0 mg |
| Komponente B, mikronisiert | 10,0 mg |
| Polyäthylenglykol 6000 | 52,0 mg |
| Kolloidale Kieselsäure | 5,0 mg |
| Stearinsäure | 3,0 mg |
| | 170,0 mg |

Herstellung von 10 000 Kernen

1,1 kg eines gleichmäßigen Gemisches der Komponenten A und B im Gewichtsverhältnis 10 : 1, mikronisiert, wird mit einer Schmelze von 0,52 kg Polyäthylenglykol [bereitet unter Zugabe von 0,05 kg kolloidaler Kieselsäure (spezifische Oberfläche 200 $m^2/g$)] vermischt und nach dem Erkalten durch ein Sieb von 1 mm Maschenbreite gedrückt. Dem Granulat werden 0,03 kg pulverförmiger, im voraus gesiebter Stearinsäure zugemischt und zu schwach gewölbten Tabletten à 170 mg verpreßt.

Herstellung von 30 000 Filmdragées

4,8 kg Kerne werden in einem Dragierkessel von 45 cm Durchmesser mit einer Lösung von Hydroxypropylmethylcellulose (Viskosität 6 cP, 2%ige Lösung in Wasser) in destilliertem Wasser, in dem 2% Talk suspendiert ist, kontinuierlich unter Warmluftzufuhr von 35° besprüht bis auf jedem Kern 5 mg Lack vorhanden ist.

Als Komponente A wird 19,21-Dihydroxypregna-4,6-dien-3,20-dion, als Komponente B 2-Phenoxy-3-[3-(1-pyrrolyl)-propyl]-5-carboxybenzolsulfonamid verwendet.

In analoger Weise kann man auch in den entsprechenden Mengen die folgenden Wirkstoffe einsetzen:

Als Komponente A:
19,21-Dihydroxypregna-4,6-dien-3,20-dion-19-acetat, -21-acetat oder -19,21-diacetat (jeweils 100 mg);
als Komponente B:
6-Chlor-7-sulfamyl-3,4-dihydro-1,2,4-benzothiadiazin-1,1-dioxid (25 mg),
2-Chlor-5-(3-hydroxy-1-oxo-isoindolyl-3)-benzolsulfonamid (50 mg),
4-(2-Methylenbutyryl)-2,3-dichlorphenoxyessigsäure (50 mg),
2-Chlor-4-furfurylamino-5-carboxybenzolsulfonamid (20 mg),
2-Phenoxy-4-butylamino-5-carboxy-benzolsulfonamid (0,5 mg) oder
3-Cyclopentylmethyl-6-chlor-7-sulfamyl-3,4-dihydro-1,2,4-benzothiadiazin-1,1-dioxid (0,5 mg).

Beispiel 5

Dragées, enthaltend 40 mg 19,21-Dihydroxy-pregna-4,6-dien-3,20-dion als Komponente A und 10 mg 6-Chlor-7-sulfamyl-3,4-dihydro-1,2,4-benzothiadiazin-1,1-dioxyd als Komponente B.

Zusammensetzung eines Dragées:

Kern:

| | |
|---|---|
| 19,21-Dihydroxypregna-4,6-dien-3,20-dion | 40 mg |
| Milchzucker | 160 mg |
| Stearylalkohol | 77 mg |
| Polyvinylpyrrolidon | 20 mg |
| Magnesiumstearat | 3 mg |
| | 300 mg |

Schutzlack

Hülle:

| | |
|---|---|
| 6-Chlor-7-sulfamyl-3,4-dihydro-1,2,4-benzothiadiazin-1,1-dioxid | 10 mg |
| Zucker, Talk, Farbstoff, Bindemittel q.s. ad | 190 mg |
| | 500 mg |

Herstellung

Die Steroid-Komponente und Milchzucker werden mit der Stearylalkoholschmelze und einer konzentrierten Polyvinylpyrrolidonlösung granuliert und getrocknet. Die erhaltene Masse wird gesiebt und zu Preßlingen von 300 mg Gewicht verpreßt. Diese werden mit einer Schutzlackschicht überzogen und sodann mit gefärbtem Zuckersirup, in welchem die diuretische Komponente gelöst ist, bis zu einem Endgewicht von ca. 500 mg dragiert.

Beispiel 6

Gelatinekapseln, enthaltend ca. 50 mg 19,21-Dihydroxypregna-4,6-dien-3,20-dion als Komponente A und 125 mg 4-Theonyl-2,3-dichlorphenoxyessigsäure als Komponente B werden folgendermaßen hergestellt:

Zusammensetzung einer Trockenkapsel:

| | |
|---|---|
| 19,21-Dihydroxypregna-4,6-dien-3,20-dion | 50,0 mg |
| 4-Thenoyl-2,3-dichlorphenoxyessigsäure | 125,0 mg |
| Milchzucker | 124,0 mg |
| Magnesiumstearat | 1,0 mg |
| | 350,0 mg |

Herstellung von 10 000 Trockenkapseln

0,50 kg 19,21-Dihydroxypregna-4,6-dien-3,20-dion, feinst gemahlen, wird mit 1,25 kg 4-Theonyl-2,3-dichlorphenoxyessigsäure innig vermischt und nach Bedarf noch zerrieben, zu diesem Gemisch 1,24 kg feinst gemahlener Laktose und 0,01 kg Magnesiumstearat durch ein Sieb zugegeben, und homogenisiert. Das Pulver wird gesiebt und in Portionen zu je 350 mg in Gelatinekapseln trocken abgefüllt.

In analoger Weise, wie in den vorangehenden Beispielen 1–6 gezeigt wurde, können auch die anderen in der Beschreibung genannten oder erwähnten Wirkstoffe zu analogen Arzneimitteln verarbeitet werden.

**Patentansprüche für die Vertragsstaaten BE, CH, DE, FR, GB, IT, LU, NL, SE**

1. Arzneimittel bzw. pharmazeutische Zusammensetzung auf der Basis von Aldosteronantagonisten und Diuretica, dadurch gekennzeichnet, daß als die Aldosteron-antagonisierende Komponente A mindestens eine 19-oxygenierte Steroid-Verbindung der Pregnan-Reihe der Formel I

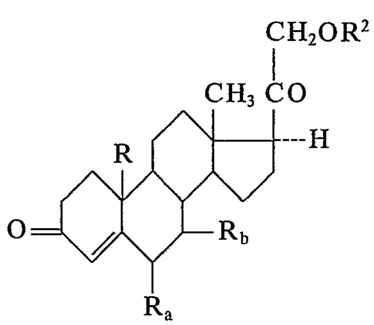

(I)

worin $R_a$ ein Wasserstoffatom und $R_b$ eine $\alpha$-orientierte $C_1-C_4$-Alkanoylthiogruppe bedeutet, oder $R_a$ und $R_b$ zusammen für eine Kohlenstoff-Kohlenstoff-Bindung stehen, R eine freie oder durch ein gerades $C_1-C_4$-Alkyl verätherte oder durch ein $C_1-C_7$-Alkanoyl veresterte Hydroxymethylgruppe

darstellt und $R^2$ ein Wasserstoffatom oder den Acylrest Ac einer Carbonsäure mit 1 – 8 C-Atomen bedeutet, und mindestens eine in bezug auf die Elektrolytausscheidung unspezifische diuretische Komponente B, im Verhältnis von 4 : 1 bis 1 : 4 (bezogen auf die jeweilige mittlere effektive Dosis), und gegebenenfalls auch pharmazeutische Träger- und/oder Hilfsstoffe enthalten sind.

2. Arzneimittel gemäß Anspruch 1 in Form von Dosierungseinheiten.

3. Mittel gemäß einem der Ansprüche 1 oder 2, dadurch gekennzeichnet, daß man als Komponente A 19,21-Dihydroxypregna-4,6-dien-3,20-dion, sein 19,21-Diacetat, 19-Acetat oder 21-Acetat verwendet.

4. Mittel gemäß einem der Ansprüche 1 – 3, dadurch gekennzeichnet, daß man als Komponente B mindestens ein diuretisch wirksames Derivat von Benzothiadiazin, Benzolsulfonamid, Phenoxyessigsäure, Benzofuran-2-carbonsäure oder 2,3-Dihydrobenzofuran-2-carbonsäure verwendet.

5. Mittel gemäß Anspruch 4, dadurch gekennzeichnet, daß man als Komponente B 1-Oxo-3-(3-sulfamyl-4-chlorphenyl)-3-hydroxyisoindolin, 6-Chloro-7-sulfamyl-3,4-dihydro-1,2,4-benzothiadiazin-1,1-dioxid, 3-Cyclopentylmethyl-6-chloro-7-sulfamyl-3,4-dihydro-1,2,4-benzothiadiazin-1,1-dioxid, 4-(2-Methylenbutyryl)-2,3-dichlorphenoxyessigsäure, 4-Thenoyl-2,3-dichlor-phenoxyessigsäure, (1-Oxo-2-methyl-2-phenyl-6,7-dichlor-5-indanyloxy)-essigsäure, 2-Chlor-4-furfurylamino-5-carboxybenzolsulfonamid, 2-Phenoxy-3-butylamino-5-carboxybenzolsulfonamid oder 2-Phenoxy-3-[3-(1-pyrrolyl)-propyl]-5-carboxybenzolsulfonamid verwendet.

6. Verfahren zur Herstellung eines Arzneimittels gemäß einem der Ansprüche 1 – 5, dadurch gekennzeichnet, daß man die in diesen Ansprüchen charakterisierten Komponenten A und B und gegebenenfalls auch die übrigen dort genannten Bestandteile in an sich bekannter Weise miteinander vermischt.

7. Verfahren gemäß Anspruch 6, dadurch gekennzeichnet, daß man ein nach diesem Anspruch hergestelltes Gemisch der Komponenten A und B und gegebenenfalls auch der übrigen dort genannten Bestandteile in eine pharmazeutische Dosierungsform in an sich bekannter Weise verarbeitet.

8. Arzneimittel gemäß einem der Ansprüche 1 – 5 zur Anwendung bei der medizinischen Behandlung bei krankhaften Zuständen bei Warmblütern, welche mit einer gestörten Ausscheidung von Harn oder Harnbestandteilen einhergehen.

9. Arzneimittel gemäß Anspruch 8 zur Verabreichung an Menschen.

**Patentansprüche für den Vertragsstaat AT**

1. Verfahren zur Herstellung von Arzneimitteln bzw. pharmazeutischen Zusammensetzungen auf der Basis von Aldosteronantagonisten und Diuretica, dadurch gekennzeichnet, daß man mindestens eine, als Aldosteron-antagonisierende Komponente A wirkende, 19-oxygenierte Steroid-Verbindung der Pregnan-Reihe der Formel I

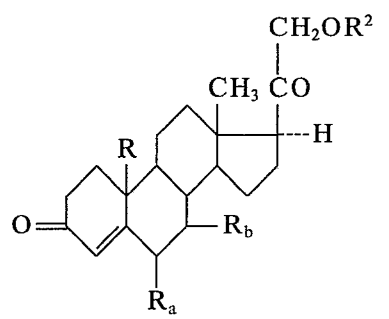

(I)

worin $R_a$ ein Wasserstoffatom und $R_b$ eine $\alpha$-orientierte $C_1 - C_4$-Alkanoylthiogruppe bedeutet, oder $R_a$ und $R_b$ zusammen für eine Kohlenstoff-Kohlenstoff-Bindung stehen, R eine freie oder durch ein gerades $C_1 - C_4$-Alkyl verätherte oder durch ein $C_1 - C_7$-Alkanoyl veresterte Hydroxymethylgruppe darstellt und $R^2$ ein Wasserstoffatom oder den Acylrest Ac einer Carbonsäure mit 1 – 8 C-Atomen bedeutet, und mindestens eine in bezug auf die Elektrolytausscheidung unspezifische diuretische Komponente B, im Verhältnis von 4 : 1 bis 1 : 4 (bezogen auf die jeweilige mittlere effektive Dosis), und gegebenenfalls auch pharmazeutische Träger- und/oder Hilfsstoffe auf nichtchemischem Wege vermischt.

2. Verfahren gemäß Anspruch 1, dadurch gekennzeichnet, daß man ein nach diesem Anspruch hergestelltes Gemisch der Komponenten A und B und gegebenenfalls auch der übrigen dort genannten Bestandteile in eine pharmazeutische Dosierungsform in an sich bekannter Weise verarbeitet.

3. Verfahren gemäß einem der Ansprüche 1 oder 2, dadurch gekennzeichnet, daß man als Komponente A 19,21-Dihydroxypregna-4,6-dien-3,20-dion, sein 19,21-Diacetat, 19-Acetat oder 21-Acetat verwendet.

4. Verfahren gemäß einem der Ansprüche 1—3, dadurch gekennzeichnet, daß man als Komponente B mindestens ein diuretisch wirksames Derivat von Benzothiadiazin, Benzolsulfonamid, Phenoxyessigsäure, Benzofuran-2-carbonsäure oder 2,3-Dihydrobenzofuran-2-carbonsäure verwendet.

5. Verfahren gemäß Anspruch 4, dadurch gekennzeichnet, daß man als Komponente B 1-Oxo-3-(3-sulfamyl-4-chlorphenyl)-3-hydroxyisoindolin, 6-Chloro-7-sulfamyl-3,4-dihydro-1,2,4-benzothiadiazin-1,1-dioxid, 3-Cyclopentylmethyl-6-chloro-7-sulfamyl-3,4-dihydro-1,2,4-benzothiadiazin-1,1-dioxid, 4-(2-Methylenbutyryl)-2,3-dichlorphenoxyessigsäure, 4-Thenoyl-2,3-dichlor-phenoxyessigsäure, (1-Oxo-2-methyl-2-phenyl-6,7-dichlor-5-indanyloxy)-essigsäure, 2-Chlor-4-furfurylamino-5-carboxybenzolsulfonamid, 2-Phenoxy-3-butylamino-5-carboxybenzolsulfonamid oder 2-Phenoxy-3-[3-(1-pyrrolyl)-propyl]-5-carboxybenzolsulfonamid verwendet.

6. Verfahren gemäß einem der Ansprüche 1—5, dadurch gekennzeichnet, daß man Arzneimittel zur Anwendung bei der medizinischen Behandlung von krankhaften Zuständen bei Warmblütern, welche mit einer gestörten Ausscheidung von Harn oder Harnbestandteilen einhergehen, herstellt.

7. Verfahren gemäß Anspruch 6, dadurch gekennzeichnet, daß das Arzneimittel zur Verabreichung an Menschen bestimmt ist.

**Claims for the Contracting States: BE, CH, DE, FR, GB, IT, LU, NL, SE**

1. A medicament or pharmaceutical composition based on aldosterone antagonists and diuretics and containing, as aldosterone-antagonising component A, at least one 19-oxygenated steroid compound of the pregnane series of the formula

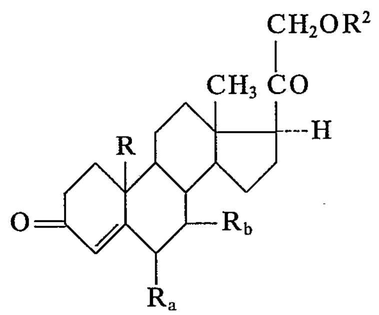

(I)

wherein

$R_a$ is a hydrogen atom, and
$R_b$ is an $\alpha$-oriented $C_1-C_4$alkanoylthio group, or
$R_a$ and $R_b$ together are a carbon-carbon bond,
R is a free hydroxymethyl group or a hydroxymethyl group etherified by a linear $C_1-C_4$alkyl or esterified by a $C_1-C_7$alkanoyl, and
$R^2$ is a hydrogen atom or the acyl radical Ac of a carboxylic acid containing 1 to 8 carbon atoms,

and at least one diuretic component B which is unspecific in respect of electrolyte excretion in the ratio 4 : 1 to 1 : 4 (based on the respective average effective dose), optionally together with pharmaceutical carriers and/or excipients.

2. A medicament according to claim 1 in dosage unit form.

3. A composition according to either of claims 1 or 2, wherein component A is 19,21-dihydroxypregna-4,6-diene-3,20-dione or the 19,21-acetate, 19-acetate or 21-acetate thereof.

4. A medicament according to any one of claims 1 to 3, which contains, as component B, at least one diuretically active derivative of benzothiadiazine, benzenesulfonamide, phenoxyacetic acid, benzofuran-2-carboxylic acid or 2,3-dihydrobenzofuran-2-carboxylic acid.

5. A medicament according to claim 4, wherein component B is 1-oxo-3-(3-sulfamyl-4-chlorophenyl)-3-hydroxyisoindoline, 6-chloro-7-sulfamyl-3,4-dihydro-1,2,4-benzothiadiazine 1,1-dioxide, 3-cyclopentylmethyl-6-chloro-7-sulfamyl-3,4-dihydro-1,2,4-benzothiadiazine 1,1-dioxide, 4-(2-methylenebutyryl)-2,3-dichlorophenoxyacetic acid, 4-thenoyl-2,3-dichlorophenoxyacetic acid, (1-oxo-2-methyl-2-phenyl-6,7-dichloro-5-indanyloxy)-acetic acid, 2-chloro-4-furfurylamino-5-carboxybenzenesulfonamide, 2-phenoxy-3-butylamino-5-carboxybenzenesulfonamide or 2-phenoxy-3-[1-(pyrrolyl)-propyl]-5-carboxybenzenesulfonamide.

6. A process for the manufacture of a medicament according to claim 1 to 5, which comprises mixing the components A and B, and optionally also the other constituents, in a manner which is known per se.

7. A process according to claim 6, which comprises compounding a mixture of components A and B as prepared therein, and optionally also the other constituents, to a pharmaceutical dosage form, in a manner which is known per se.

8. A medicament according to any one of claims 1 to 5 for use in the medical treatment of pathological conditions in warm-blooded animals, which conditions are consequent on an impaired excretion of urine or urinary constituents.

9. A medicament according to claim 8 for administration to man.

**Claims for the Contracting Stade: AT**

1. A process for the production of a medicament or pharmaceutical composition based on aldosterone antagonists and diuretics, which comprises mixing, as aldosterone-antagonising component A, at least one 19-oxygenated steroid compound of the pregnane series of the formula

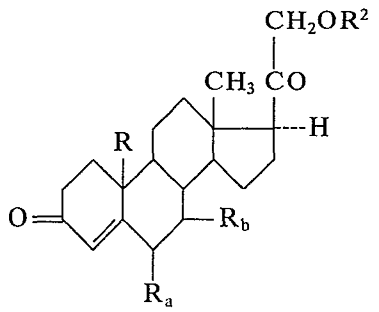

(I)

wherein

$R_a$ is a hydrogen atom, and
$R_b$ is an $\alpha$-oriented $C_1-C_4$alkanoylthio group, or
$R_a$ and $R_b$ together are a carbon-carbon bond,
R is a free hydroxymethyl group or a hydroxymethyl group etherified by a linear $C_1-C_4$alkyl or esterified by a $C_1-C_7$alkanoyl, and
$R^2$ is a hydrogen atom or the acyl radical Ac of a carboxylic acid containing 1 to 8 carbon atoms,

and at least one diuretic component B which is unspecific in respect of electrolyte excretion in the ratio from 4 : 1 to 1 : 4 (based on the respective average effective dose), and optionally also pharmaceutical carriers and/or excipients, by a non-chemical method.

2. A process according to claim 1, which comprises compounding a mixture of components A and B as prepared therein, and optionally also the other constituents, to a pharmaceutical dosage form, in a manner which is known per se.

3. A process according to either of claims 1 or 2, wherein component A is 19,21-dihydroxypregna-4,6-diene-3,20-dione or the 19,21-acetate, 19-acetate or 21-acetate thereof.

4. A process according to any one of claims 1 to 3, which comprises using, as component B, at least one diuretically active derivative of benzothiadiazine, benzenesulfonamide, phenoxyacetic acid, benzofuran-2-carboxylic acid or 2,3-dihydrobenzofuran-2-carboxylic acid.

5. A process according to claim 4, wherein component B is 1-oxo-3-(3-sulfamyl-4-chlorophenyl)-3-hydroxyisoindoline, 6-chloro-7-sulfamyl-3,4-dihydro-1,2,4-benzothiadiazine 1,1-dioxide, 3-cyclopentylmethyl-6-chloro-7-sulfamyl-3,4-dihydro-1,2,4-benzothiadiazine 1,1-dioxide, 4-(2-methylenebutyryl)-2,3-dichlorophenoxyacetic acid, 4-thenoyl-2,3-dichlorophenoxyacetic acid, (1-oxo-2-methyl-2-phenyl-6,7-dichloro-5-indanyloxy)-acetic acid, 2-chloro-4-furfurylamino-5-carboxybenzenesulfonamide, 2-phenoxy-3-butylamino-5-carboxybenzenesulfonamide or 2-phenoxy-3-[3-(1-pyrrolyl)-propyl]-5-carboxybenzenesulfonamide.

6. A process according to any one of claims 1 to 5 for the preparation of a medicament for use in the medical treatment of pathological conditions in a warm-blooded animal which are consequent on an impaired excretion of urine or urinary constituents.

7. A process according to claim 6, wherein the medicament is intended for administration to man.

**Revendications pour les Etats contractants: BE, CH, DE, FR, GB, IT, LU, NL, SE**

1. Médicaments ou compositions pharmaceutiques à base d'antagonistes de l'aldostérone et de diurétiques, caractérisés en ce qu'ils contiennent comme composant A antagoniste de l'aldostérone au moins un composé stéroïdique oxygéné en 19 de la série des pregnanes de formule

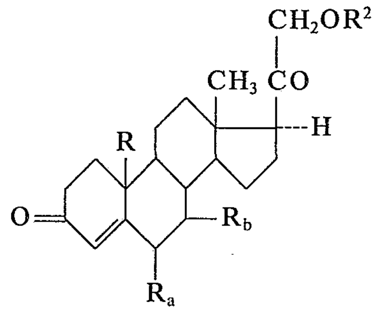

(I)

où $R_a$ représente un atome d'hydrogène et $R_b$ un groupe alcanoylthio en $C_1$ à $C_4$ orienté en alpha, ou bien où $R_a$ et $R_b$ représentent ensemble une liaison carbone-carbone, R un groupe hydroxyméthyle libre ou éthérifié par un alcoyle en $C_1$ à $C_4$ à chaîne droite ou estérifié par un alcanoyle en $C_1$ à $C_7$ et $R_2$ représente un atome d'hydrogène ou le radical acyle Ac d'un acide carboxlique en $C_1$ à $C_8$, et au moins un composant diurétique B non spécifique par rapport à l'excrétion d'électrolytes, dans un rapport de 4 : 1 à 1 : 4 (par rapport à la dose efficace moyenne respective), et éventuellement aussi des supports et/ou additifs pharmaceutiques.

2. Médicaments selon la revendication 1 sous forme d'unités posologiques.

3. Agent selon l'une des revendications 1 ou 2, caractérisé en ce qu'on utilise comme composant A la 19,21-dihydroxy-pregna-4,6-diène-3,20-dione, son 19,21-diacétate, son 19-acétate ou son 21-acétate.

4. Agent selon l'une des revendications 1—3, caractérisé en ce qu'on utilise comme composant B au moins un dérivé à action diurétique de la benzothiadiazine, du benzènesulfonamide, de l'acide phénoxyacétique, de l'acide benzofuranne-2-carboxylique ou de l'acide 2,3-dibenzofuranne-2-carboxylique.

5. Agent selon la revendication 4, caractérisé en ce qu'on utilise comme composant B la 1-oxo-3-(3-sulfamyl-4-chlorophényl)-3-hydroxyisoindoline, le 6-chloro-7-sulfamyl-3,4-dihydro-1,2,4-benzothiadiazin-1,1-dioxyde, le 3-cyclopentylméthyl-6-chloro-7-sulfamyl-3,4-dihydro-1,2,4-benzothiadiazin-1,1-dioxyde, l'acide 4-(2-méthylènebutyryl)-2,3-dichlorophénoxyacétique, l'acide 4-thénoyl-2,3-dichloro-phénoxyacétique, l'acide (1-oxo-2-méthyl-2-phényl-6,7-dichloro-5-indanyloxy)-acétique, le 2-chloro-4-furfurylamino-5-carboxybenzènesulfonamide, le 2-phénoxy-3-butylamino-5-carboxybenzènesulfonamide ou le 2-phénoxy-3-[3-(1-pyrrolyl)-propyl]-5-carboxybenzènesulfonamide.

6. Procédé de préparation d'un médicament selon l'une des revendications 1—5, caractérisé en ce qu'on mélange de façon classique les composants A et B caractérisés dans les présentes revendications et éventuellement également les composants qui y sont mentionnés.

7. Procédé selon la revendication 6, caractérisé en ce qu'on traite de façon classique un mélange des composants A et B et éventuellement également des autres composants qui sont mentionnés dans cette revendication, préparé selon cette revendication, pour obtenir une forme posologique pharmaceutique.

8. Médicament selon l'une des revendications 1—5 aux fins d'application dans le traitement médical des états maladifs comportant un désordre de l'excrétion de l'urée ou des composants de l'urée chez les homéothermes.

9. Médicament selon la revendication 8 aux fins d'administration chez l'homme.

**Revendications pour l'Etat contractant: AT**

1. Procédé de préparation de médicaments ou de compositions pharmaceutiques à base d'antagonistes de l'aldostérone et de diurétiques, caractérisé en ce qu'on mélange par un moyen non chimique comme composant A à action antagoniste de l'aldostérone, au moins un composé stéroidique oxygéné en 19 de la série des pregnanes de formule

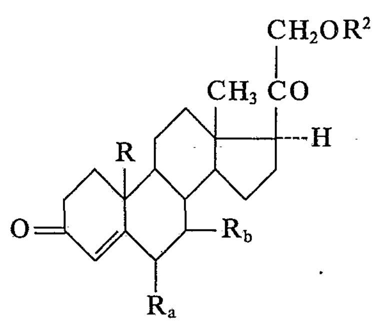

(I)

où $R_a$ représente un atome d'hydrogène et $R_b$ un groupe alcanoylthio en $C_1$ à $C_4$ orienté en alpha, ou bien où $R_a$ et $R_b$ représentent ensemble une liaison carbone-carbone, R représente un groupe hydroxyméthyle libre ou éthérifié par un alcoyle en $C_1$ à $C_4$ à chaîne droite ou estérifié par un alcanoyle en $C_1$ à $C_7$ et $R_2$ un atome d'hydrogène ou le radical acyle Ac d'un acide carboxylique en $C_1$ à $C_8$, et au moins un composant diurétique B non spécifique vis-à-vis de l'excrétion des électrolytes, dans un rapport de 4 : 1 à 1 : 4 (par rapport à la dose efficace movenne respective), et éventuellement également des supports et/ou additifs pharmaceutiques.

2. Procédé selon la revendication 1, caractérisé en ce qu'on traite de façon classique un mélange des composants A et B et éventuellement également des autres composants qui y sont mentionnés, préparé selon cette revendication, pour obtenir une forme posologique pharmaceutique.

3. Procédé selon l'une des revendications 1 ou 2, caractérisé en ce qu'on utilise comme composant A la 19,21-dihydroxy-pregna-4,6-diène-3,20-dione, son 19,21-diacétate, 19-acétate ou 21-acétate.

4. Procédé selon l'une des revendications 1—3, caractérisé en ce qu'on utilise comme composant B au moins un dérivé à action diurétique de la benzothiadiazine, du benzènesulfonamide, de l'acide phénoxyacétique, de l'acide benzofuranne-2-carboxylique ou de l'acide 2,3-dihydrobenzofuranne-2-carboxylique.

5. Procédé selon la revendication 4, caractérisé en ce qu'on utilise comme composant B la 1-oxo-3-(3-sulfamyl-4-chlorophényl)-3-hydroxyisoindoline, le 6-chloro-7-sulfamyl-3,4-dihydro-1,2,4-benzothiadiazin-1,1-dioxyde, le 3-cyclopentylméthyl-6-chloro-7-sulfamyl-3,4-dihydro-1,2,4-benzothiadiazin-1,1-dioxyde, l'acide 4-(2-méthylènebutyryl)-2,3-dichlorophénoxyacétique, l'acide 4-thénoyl-2,3-dichloro-phénoxyacétique, l'acide (1-oxo-2-méthyl-2-méthyl-2-phényl-6,7-dichloro-5-indanyloxy)-acétique, le 2-chloro-4-furfurylamino-5-carboxybenzènesulfonamide, le 2-phénoxy-3-butylamino-5-carboxybenzènesulfonamide ou le 2-phénoxy-3-[3-(1-pyrrolyl)-propyl]-5-carboxybenzènesulfonamide.

6. Procédé selon l'une des revendications 1—5, caractérisé en ce qu'on prépare des médicaments aux fins d'application dans le traitement médical des états maladifs qui se manifestent par un désordre de l'excrétion de l'urée ou des composants de l'urée chez les homéothermes.

7. Procédé selon la revendication 6, caractérisé en ce que le médicament est déterminé en vue de l'administration chez l'homme.